(19) Europäisches Patentamt
European Patent Office
Office européen des brevets

(11) Veröffentlichungsnummer: **0 296 495 B1**

(12) # EUROPÄISCHE PATENTSCHRIFT

(45) Veröffentlichungstag der Patentschrift: **04.03.92**

(51) Int. Cl.5: **C07C 209/60**, C07C 211/09, B01J 29/04, B01J 29/08, B01J 29/28

(21) Anmeldenummer: **88109641.6**

(22) Anmeldetag: **16.06.88**

(54) **Verfahren zur Herstellung von Diaminen.**

(30) Priorität: **23.06.87 DE 3720676**

(43) Veröffentlichungstag der Anmeldung:
**28.12.88 Patentblatt 88/52**

(45) Bekanntmachung des Hinweises auf die Patenterteilung:
**04.03.92 Patentblatt 92/10**

(84) Benannte Vertragsstaaten:
**BE CH DE FR GB LI NL**

(56) Entgegenhaltungen:
**EP-A- 0 132 736**
**EP-A- 0 133 938**
**DE-A- 2 338 419**
**FR-A- 2 476 642**

**PATENT ABSTRACTS OF JAPAN, Band 3, Nr. 50 (C-44), 27.4.1979; & JP-A-54 024836**

**PATENT ABSTRACTS OF JAPAN, Band 5, Nr. 71, (C-54)(743), 13.5.1981; & JP-A-56 020552**

(73) Patentinhaber: **BASF Aktiengesellschaft**
**Carl-Bosch-Strasse 38**
**W-6700 Ludwigshafen(DE)**

(72) Erfinder: **Hesse, Michael, Dr.**
**An der Froschlache 3**
**W-6700 Ludwigshafen(DE)**
Erfinder: **Hoelderich, Wolfgang, Dr.**
**Mannheimer Strasse 18 c**
**W-6710 Frankenthal(DE)**
Erfinder: **Schwarzmann, Matthias, Dr.**
**Carl-Bosch-Strasse 54**
**W-6703 Limburgerhof(DE)**

EP 0 296 495 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein Verfahren zur Herstellung von Diaminen durch Umsetzung von Diaminen mit Olefinen in Gegenwart von Zeolithen als Katalysatoren.

Die N-Substitution von Diaminen mit zwei primären oder sekundären Aminogruppen, speziell von Ethylendiamin,ist bekannt und kann z.B. durch Umsetzung des Diamins mit Alkylhalogeniden, mit Carbonylverbindungen unter reduzierenden Bedingungen oder durch Umsetzung von $\alpha,\omega$-Halogenaminen mit primären Aminen oder Ammoniak erfolgen. Es treten jedoch auch in hohem Maße N,N′-Disubstitutionsprodukte auf. Die Ausgangsstoffe sind wie im Fall der Halogenamine schwer zugänglich. Die Herstellung bestimmter mono-N-alkylierter Diamine ist dadurch erschwert.

Die bei den oben genannten Verfahren z. T. anfallenden starken Säuren und ihre Ammoniumsalze werfen starke Korrosionsprobleme auf. Auch ist die Verwendung von halogenhaltigen Verbindungen aus Gründen des Umweltschutzes ob ihrer Giftigkeit und der Korrosivität der freiwerdenden Stoffe, z.B. HJ, HBr oder deren Ammoniumsalze problematisch.

Es ist bekannt, Amine mit Olefinen, vorzugsweise mit an der Doppelbindung substituierten Olefinen, an sauren Ionenaustauschern (US 4 536 602) und an Zeolithen zu substituierten Aminen umzusetzen (DE 33 26 579, DE 33 27 000 und US 4 375 002, EP 39 918, EP 77 016, EP 101 921). Die so beschriebenen Verfahren erwähnen aber nur die Addition von $NH_3$ oder Monoaminen, hingegen sind Umsetzungen mit Diaminen nicht beschrieben.

Dabei ergibt sich nun die Aufgabe, ein Verfahren zu entwickeln, nach dem Diamine, bei denen die Möglichkeit einer Substitution an beiden N-Atomen besteht, selektiv zu substituieren, unter Verwendung von gut zugänglichen, wenig korrosiven Ausgangsstoffen und Katalysatoren.

Es wurde nun überraschend gefunden, daß man bei der Umsetzung von Diaminen mit Olefinen in Gegenwart von Zeolithen als Katalysatoren in guten Ausbeuten Diamine erhält, bei denen nur an einem N-Atom im Rahmen der Reaktion Substitution stattfindet.

Man erhält die o.g. N-substituierten Diamine der allgemeinen Formel (I)

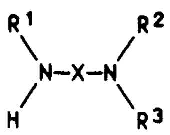

(I)

in der $R^1$, $R^2$ und $R^3$ für Wasserstoff oder eine Alkyl-, Aryl-, Alkoxy-, Aralkyl- oder Cycloalkylgruppe stehen kann und X eine Gruppierung der Formel (II) beschreibt,

$(CR^4R^5)_m$ (II)

wobei $R^4$ und $R^5$ obige Bedeutung annehmen können wie $R^1$, und m die Werte zwischen 1 und 12 annehmen kann, aus Diaminen der Formel (III)

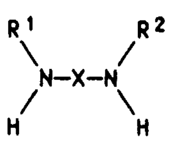

(III)

wobei $R^1$, $R^2$ und X obige Gruppen darstellen, wie in Formel (I) beschrieben, mit Olefinen der Formel (IV)

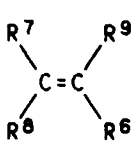

(IV)

in der die Reste $R^6$ bis $R^9$ Wasserstoff oder einen in $\alpha$-Stellung unverzweigten Alkylrest darstellen, in

Gegenwart von Zeolithen als Katalysatoren.

Dies ist umso überraschender, als Disubstitutionsprodukte nicht nachweisbar sind und dies trotz eines Überschusses an Olefin gilt.

Olefine der Formel (IV), die erfindungsgemäß als Ausgangsstoffe bevorzugt umgesetzt werden können sind z.B. Ethen, n-Propen, n-Buten, Isobuten, n-Penten, 2-Methylbutene, n-Hexen, 2-Ethylbutene, 3-Ethylbutene, n-Octen, 2-Ethylocten, Nonene, Docene, Cyclopenten, Cyclohexen, 1-Methylcyclopenten, 1-Methylcyclohexen und Cycloocten.

Beispiele für die erfindungsgemäß umgesetzten Di- und Polyamine sind z.B. Ethyldiamin, 1,3-Diaminopropan, 1,4-Diaminobutan, 1,2-Propylendiamin, 2-Ethylaminoethylamin, 3-Amino-1-methylaminopropan, 3-Amino-1-cyclohexylaminopropan, Neopentandiamin, Hexamethylendiamin, 4,4′-Diaminodicyclohexylmethan, 3,3′-Dimethyl-4,4′-diaminodiphenylmethan.

Als Katalysatoren für das erfindungsgemäße Verfahren verwendet man Zeolithe, zweckmäßig in der aciden Form, Zeolithe sind kristalline Aluminiumsilikate, die eine hochgeordnete Struktur mit einem starren dreidimensionalen Netzwerk von $SiO_4$- und $AlO_4$-Tetraedern besitzen, die durch gemeinsame Sauerstoffatome verbunden sind. Das Verhältnis der Si-und Al-Atome zu Sauerstoff beträgt 1 : 2. Die Elektrovalenz der Aluminium enthaltenden Tetraeder ist durch Einschluß von Kationen in den Kristall, z.B. eines Alkali- oder Wasserstoffions ausgeglichen. Ein Kationenaustausch ist möglich. Die Räume zwischen den Tetraedern sind vor der Dehydration durch Trocknen bzw. Calcinieren von Wassermolekülen besetzt.

In den Zeolithen können anstelle von Aluminium auch andere Elemente wie B, Ga, Fe, Cr, V, As, Sb, Bi oder Be oder deren Gemische in das Gitter eingebaut werden, oder das Silicium kann durch ein vierwertiges Element wie Ge, Ti, Zr, Hf ersetzt werden.

Entsprechend ihrer Struktur werden Zeolithe in verschiedene Gruppen unterteilt. So bilden bei der Mordenit-Gruppe Ketten oder bei der Chabasit-Gruppe Schichten aus Tetraedern die Zeolith-Struktur, während sich bei der Faujasit-Gruppe die Tetraeder zu Polyedern ordnen, z.B. in Form eines Kubooktaeders, der aus Vierringen bzw. Sechsringen aufgebaut ist. Je nach Verknüpfung der Kubooktaeder, wodurch unterschiedlich große Hohlräume und Poren entstehen, unterscheidet man in Zeolithe vom Typ A, L, X oder Y.

Für das erfindungsgemäße Verfahren in Betracht kommende Katalysatoren sind Zeolithe aus der Mordenit-Gruppe oder engporige Zeolithe vom Erionit-bzw.- Chabasit-Typ oder Zeolithe vom Faujasit-Typ, z.B. Y-, X- oder L-Zeolithe.

In diese Gruppe von Zeolithen gehören auch die sogenannten "ultrastabilen" Zeolithe des Faujasittyps, d.h. dealuminierte Zeolithe. Verfahren zur Herstellung solcher Zeolithe sind mannigfach beschrieben.

Besonders vorteilhaft sind Zeolithe vom Pentasiltyp. Diese haben als Grundbaustein einen aus $SiO_4$-Tetraedern aufgebauten Fünfring gemeinsam. Sie sind durch ein hohes $SiO_2/Al_2O_3$-Verhältnis gekennzeichnet sowie durch Porengrößen, die zwischen denen derZeolithe vom Typ A und denen vom Typ X oder Y liegen.

Diese Zeolithe können unterschiedliche chemische Zusammensetzung aufweisen. Es handelt sich hierbei um Alumino-, Boro-, Eisen-, Beryllium, Gallium-, Chrom-, Arsen-, Antimon- und Wismutsilikatzeolithe oder deren Gemische sowie Alumino-, Boro-, Gallium- und Eisengermanatzeolithe oder deren Gemische. Insbesondere eignen sich die Alumino-, Boro- und Eisensilikatzeolithe des Pentasiltyps für das erfindungsgemäße Verfahren. Der Aluminosilikatzeolith wird z.B. aus einer Aluminiumverbindung, vorzugsweise $Al(OH)_3$ oder $Al_2(SO_4)_3$ und einer Siliciumkomponente, vorzugsweise hochdispersem Siliciumdioxid in wäßriger Aminlösung, insbesondere in Polyaminen wie 1,6-Hexandiamin- oder 1,3-Propandiamin-oder Triethylentetramin-Lösung mit oder insbesondere ohne Alkali- oder Erdalkalizusatz bei 100 bis 220 °C unter autogenem Druck hergestellt. Hierzu gehören auch die isotaktischen Zeolithe nach EP 34 727 und EP 46 504. Die erhaltenen Aluminosilikatzeolithe enthalten je nach Wahl der Einsatzstoffmengen ein $SiO_2/Al_2O_3$-Verhältnis von 10 bis 40 000. Aluminosilikatzeolithe des Pentasiltyps können auch in etherischem Medium wie Diethylenglykoldimethylether, in alkoholischem Medium wie Methanol bzw. 1,4-Butanol oder in Wasser synthetisiert werden.

Zu den erfindungsgemäß verwendbaren siliciumreichen Zeolithen ($SiO_2/Al_2O_3 \geqq 10$) gehören auch die verschiedenen ZSM-Typen, Ferrierit, Nu-1 und Silicalit®.

Borosilikatzeolithe kann man z.B. bei 90 bis 200 °C unter autogenem Druck synthetisieren, indem man eine Borverbindung, z.B. $H_3BO_3$, mit einer Siliciumverbindung, vorzugsweise hochdispersem Siliciumdioxid in wäßriger Aminlösung, insbesondere in 1,6-Hexandiamin- oder 1,3-Propandiamin- oder Triethylentetraamin-Lösung mit und insbesondere ohne Alkali- oder Erdalkalizusatz zur Reaktion bringt. Hierzu gehören auch die isotaktischen Zeolithe nach EP 34 727 und EP 46 504. Solche Borosilikatzeolithe können ebenfalls hergestellt werden, wenn man die Reaktion statt in wäßriger Aminlösung in etherischer Lösung, z.B. Diethylenglykoldimethylether oder in alkoholischer Lösung, z.B. 1,6-Hexandiol durchführt.

Den Eisensilikatzeolith erhält man z.B. aus einer Eisenverbindung, vorzugsweise $Fe_2(SO_4)_3$ und einer Siliciumverbindung, vorzugsweise hochdispersem Siliciumdioxid in wäßriger Aminlösung, insbesondere 1,6-Hexandiamin, mit oder ohne Alkali- oder Erdalkalizusatz bei 100 bis 200 °C unter autogenem Druck.

Die so hergestellten Alumino-, Boro- und Eisensilikatzeolithe können nach ihrer Isolierung, Trocknung bei 100 bis 160 °C, vorzugsweise 110 °C und Calcinierung bei 450 bis 550 °C, vorzugsweise 500 °C, mit einem Bindemittel im Verhältnis 90 : 10 bis 40 : 60 Gew.-% zu Strängen oder Tabletten verformt werden. Als Bindemittel eignen sich diverse Aluminiumoxide, bevorzugt Boehmit, amorphe Aluminosilikate mit einem $SiO_2/Al_2O_3$-Verhältnis von 25 : 75 bis 90 : 5, bevorzugt 75 : 25, Siliciumdioxid, bevorzugt hochdisperses $SiO_2$, Gemische aus hochdispersem $SiO_2$ und hochdispersem $Al_2O_3$, $TiO_2$, $ZrO_2$ sowie Ton. Nach der Verformung werden die Extrudate oder Preßlinge bei 110 °C/16 h getrocknet und bei 500 °C/16 h calciniert.

Man erhält auch vorteilhafte Katalysatoren, wenn der isolierte Alumino- bzw. Borosilikatzeolith direkt nach der Trocknung verformt wird und erst nach der Verformung einer Calcinierung unterworfen wird. Die hergestellten Alumino- und Borosilikatzeolithe können in reiner Form, ohne Binder, als Stränge oder Tabletten eingesetzt werden, wobei als Verstrangungs- oder Peptisierungshilfsmittel, z.B. Ethylcellulose, Stearinsäure, Kartoffelstärke, Ameisensäure, Oxalsäure, Essigsäure, Salpetersäure, Ammoniak, Amine, Silikoester und Graphit oder deren Gemische verwendet werden. Liegt der Zeolith aufgrund der Art seiner Herstellung nicht in der katalytisch aktiven, aciden H-Form vor, sondern z.B. in der Na-Form, dann kann diese durch Ionenaustausch, z.B. mit Ammoniumionen und anschließende Calcinierung oder durch Behandlung mit Säuren vollkommen oder partiell in die gewünschte H-Form überführt werden.

Um eine möglichst hohe Selektivität, hohe Umsätze sowie lange Standzeiten zu erreichen, ist es vorteilhaft, die Zeolithe zu modifizieren. Eine geeignete Modifizierung der Katalysatoren besteht z.B. darin, daß man den unverformten oder verformten Zeolithen mit Metallsalzen durch einen Ionenaustausch oder durch Imprägnierung dotiert. Als Metalle werden Alkalimetalle wie Li, Cs, K, Erdalkalimetall wie Mg, Ca, Sr, Ba, Metalle der 3., 4. und 5. Hauptgruppe wie B, Al, Ga, Ge, Sn, Pb, Bi, seltene Erdmetalle wie La, Ce, Pr, Nd, Er, Yb und U eingesetzt. Die Modifizierung mit diesen Metallen kann durch Ionenaustausch oder Imprägnierung erfolgen.

Zweckmäßigerweise führt man diese Dotierung so durch, daß man den verformten Zeolithen in einem Steigrohr vorlegt und bei 20 bis 100 °C eine wäßrige oder ammoniakalische Lösung eines Halogenids oder eines Nitrats der voranbeschriebenen Metalle überleitet. Ein derartiger Ionenaustausch kann an der Wasserstoff-, Ammonium- und Alkaliform des Zeolithen vorgenommen werden. Eine weitere Möglichkeit der Metallaufbringung auf den Zeolithen ist gegeben, indem man das zeolithische Material, z.B. mit einem Halogenid, einem Nitrat oder einem Oxid der voranbeschriebenen Metalle in wäßriger, alkoholischer oder ammoniakalischer Lösung imprägniert. Sowohl an einen Ionenaustausch als auch an eine Imprägnierung schließt sich zumindest eine Trocknung, wahlweise eine abermalige Calcinierung an.

Eine mögliche Ausführungsform besteht z.B. darin, daß man $Cu(NO_3)_2 \times 3\ H_2O$ oder $Ni(NO_3)_2 \times 6\ H_2O$ oder $Ce(NO_3)_3 \times 6\ H_2O$ oder $Cr(NO_3)_3 \times 6\ H_2O$ oder $Pd(NO_3)_2$ in Wasser löst und mit dieser Lösung den verformten oder unverformten Zeolith eine gewisse Zeit, z.B. 30 Minuten, tränkt. Die eventuell überstehende Lösung wird im Rotationsverdampfer von Wasser befreit. Danach wird der getränkte Zeolith bei etwa 150 °C getrocknet und bei etwa 550 °C calciniert. Dieser Tränkvorgang kann mehrmals hintereinander vorgenommen werden, um den gewünschten Metallgehalt einzustellen.

Es ist auch möglich, eine wäßrige $Ni(NO_3)_2$-Lösung oder ammoniakalische $Pd(NO_3)_2$-Lösung herzustellen und darin den reinen pulverförmigen Zeolithen bei 40 bis 100 °C unter Rühren etwa 24 h aufzuschlämmen. Nach Abfiltrieren, Trocknen bei etwa 150 °C und Calcinierung bei etwa 500 °C kann das so gewonnene zeolithische Material mit oder ohne Bindemittel zu Strängen, Pellets oder Wirbelgut weiterverarbeitet werden.

Ein Ionenaustausch des in der H-Form oder Ammonium-Form oder Alkali-Form vorliegenden Zeolithen kann so vorgenommen werden, daß man den Zeolithen in Strängen oder Pellets in einer Kolonne vorlegt und darüber eine wäßrige $Ni(NO_3)_2$-Lösung oder ammoniakalische $Pd(NO_3)_2$-Lösung bei leicht erhöhter Temperatur zwischen 30 und 80 °C im Kreislauf 15 bis 20 h leitet. Danach wird mit Wasser ausgewaschen, bei etwa 150 °C getrocknet und bei etwa 550 °C calciniert.

Bei manchen metalldotierten Zeolithen, z.B. Pd-, Cu-, Ni-dotierten Zeolithen ist eine Nachbehandlung mit Wasserstoff vorteilhaft.

Eine weitere Möglichkeit der Modifizierung besteht darin, daß man das zeolithische Material - verformt oder unverformt - einer Behandlung mit Säuren wie Salzsäure, Flußsäure und Phosphorsäure und/oder Wasserdampf unterwirft.

Im einzelnen geht man vorteilhaft so vor, daß man Zeolithe in Pulverform mit 1 n Phosphorsäure 1 Stunde bei 80 °C behandelt. Nach der Behandlung wird mit Wasser gewaschen, bei 110 °C/16 Stunden getrocknet und bei 500 °C/20 Stunden calciniert. Nach einer anderen Arbeitsweise behandelt man Zeolithe

vor oder nach ihrer Verformung mit Bindemittel, z.B. 1 bis 3 Stunden bei Temperaturen von 60 bis 80 °C mit einer 3 bis 25 Gew.-%igen, insbesondere 12 bis 20 Gew.-%igen wäßrigen Salzsäure. Anschließend wird der so behandelte Zeolith mit Wasser gewaschen, getrocknet und bei 400 °C bis 500 °C calciniert.

Eine besondere Ausführungsform für die Säurebehandlung besteht darin, daß man das zeolithische Material vor seiner Verformung bei erhöhter Temperatur mit Flußsäure, die im allgemeinen als 0,001 n bis 2 n, vorzugsweise 0,05 n bis 0,5 n Flußsäure eingesetzt wird, behandelt, beispielsweise durch Erhitzen unter Rückfluß über einen Zeitraum von 0,5 bis 5, vorzugsweise 1 bis 3 h. Nach Isolierung, durch Abfiltrieren und Auswaschen des zeolithischen Materials, wird dieses zweckmäßig, bei Temperaturen von 100 bis 160 °C, getrocknet und bei Temperaturen von 450 °C bis 600 °C calciniert. Gemäß einer weiteren bevorzugten Ausführungsform für die Säurebehandlung, wird das zeolithische Material nach einer Verformung mit Bindemittel bei erhöhter Temperatur, zweckmäßig bei Temperaturen von 50 bis 90 °C, vorzugsweise 60 bis 80 °C, über einen Zeitraum von 0,5 bis 5 h, vorzugsweise mit 12 bis 20 Gew.-%iger Salzsäure, behandelt. Das zeolithische Material kann anschließend ausgewaschen, bei Temperaturen von 100 bis 160 °C getrocknet und bei Temperaturen von 450 bis 600 °C calciniert werden. Einer HF-Behandlung kann sich auch eine HCl-Behandlung anschließen.

Nach einer anderen Arbeitsweise kann man Zeolithe durch Aufbringen von Phosphorverbindungen, wie Trimethoxiphosphat, Trimethoxiphosphin, primärem, sekundärem oder tertiärem Natriumphosphat modifizieren. Besonders vorteilhaft hat sich die Behandlung mit primärem Natriumphosphat erwiesen. Hierbei werden die Zeolithe in Strang-, Tabletten- oder Wirbelgut-Form mit wäßriger $NaH_2PO_4$-Lösung getränkt, bei 110 °C getrocknet und bei 500 °C calciniert.

Wenn bei der erfindungsgemäßen Verwendung der zeolithischen Katalysatoren eine durch Koksabscheidung bedingte Desaktivierung eintritt, empfiehlt es sich, diese durch Abbrennen der Koksablagerung mit Luft oder mit einem Luft/$N_2$-Gemisch bei 400 bis 550 °C, bevorzugt 500 °C, zu regenerieren. Die Zeolithe erhalten dadurch ihre Anfangsaktivität zurück.

Durch partielle Verkokung (pre-coke) ist es möglich, die Aktivität des Katalysators für ein Selektivitätsoptimum des gewünschten Reaktionsproduktes einzustellen.

Die hier beschriebenen Katalysatoren können wahlweise als 2- bis 4-mm-Stränge oder als Tabletten mit 3 bis 5 mm Durchmesser oder als Splitt mit Teilchengrößen von 0,1 bis 0,5 mm oder als Wirbelkontakt eingesetzt werden.

Die für die erfindungsgemäße Umsetzung in der Regel gewählten Reaktionsbedingungen sind 50 bis 500 °C, z.B. 150 bis 450 °C und insbesondere 200 bis 400 °C und eine Katalysatorbelastung WHSV von 0,1 bis 20 $h^{-1}$, insbesondere von 1,0 bis 10,0 $h^{-1}$ g Edukt je g Katalysator und Stunde.

Die Umsetzung wird im allgemeinen bei einem Druck zwischen 50 und 500 bar durchgeführt, insbesondere zwischen 150 und 350 bar.

Das Molverhältnis der Einsatzstoffe Diamin/Olefin liegt in der Regel zwischen 10 : 1 und 1 : 10, vorzugsweise zwischen 3 : 1 und 1 : 2.

Das Verfahren wird im allgemeinen diskontinuierlich, vorzugsweise kontinuierlich durchgeführt.

Schwerflüchtige oder feste Ausgangsstoffe kann man in gelöster Form, z.B. in THF-, Toluol- oder Petrolether-Lösung einsetzen. Es ist auch eine Verdünnung des Ausgangsstoffes mit derartigen Lösungsmitteln möglich.

Nach der Umsetzung werden die entstandenen Produkte durch übliche Verfahren, z.B. durch Destillation oder Extraktion, aus dem Reaktionsgemisch isoliert und nötigenfalls mittels weiterer Trennoperationen auf die gewünschte Reinheit gebracht; nichtumgesetzte Ausgangsstoffe können in den Reaktor zurückgeführt werden.

Beispiele 1 bis 7

Für die Beispiele werden folgende Katalysatoren eingesetzt:

Katalysator A

Ein Borosilikatzeolith des Pentasil-Typs wird in einer hydrothermalen Synthese aus 640 g hochdispersem $SiO_2$, 122 g $H_3BO_3$, 8000 g einer wäßrigen 1,6-Hexandiamin-Lösung (Mischung 50 : 50 Gew.-%) bei 170 °C unter autogenem Druck in einem Rührautoklaven hergestellt. Nach Abfiltrieren und Auswaschen wird das kristalline Reaktionsprodukt bei 100 °C/24 h getrocknet und bei 500 °C/24 h calciniert. Dieser Borosilikatzeolith setzt sich zusammen aus 94,2 Gew.-% $SiO_2$ und 2,3 Gew.-% $B_2O_3$. Daraus werden durch Verformen mit Boehmit im Gewichtsverhältnis 60 : 40 2 mm-Stränge hergestellt, die bei 110 °C/16 h getrocknet und bei 500 °C/24 h calciniert werden.

Katalysator B

Katalysator B erhält man, indem man Katalysator A mit einer wäßrigen $Cr(NO_3)_3$-Lösung imprägniert danach bei 130 °C/2 h trocknet und bei 500 °C/24 h calciniert. Der Cr-Gehalt beträgt 3,2 Gew.-%.

Katalysator C

Katalysator C wird wie Katalysator B hergestellt, jedoch mit einer wäßrigen Lösung aus Ce-Nitrat statt Cr-Nitrat getränkt. Der Ce-Gehalt beträgt 7,1 Gew.-%.

Katalysator D

Katalysator D wird entsprechend Katalysator B hergestellt, jedoch mit einer wäßrigen Lösung aus Pb-Nitrat statt Cr-Nitrat getränkt. Der Pb-Gehalt beträgt 1,2 Gew.-%.

Beispiele 1 und 2

In einem 0,3 l-Rührautoklaven werden jeweils 10 ml des oben beschriebenen Katalysators A gegeben und das Diamin eingefüllt. Nach dem Verschließen werden die Olefine soweit gasförmig, aufgedrückt, ansonsten werden sie zusammen mit dem Diamin eingefüllt. Die Menge an Einsatzprodukt wird so bemessen, daß bei der gewählten Reaktionstemperatur der gewünschte Druck als Eigendruck erreicht wird. Ist das nicht möglich, wird mit Stickstoff der gewünschte Druck eingestellt.

Beispiele 3 bis 7

Die Reaktion wird unter isothermen Bedingungen in einem Rohrreaktor (Wendel, 0,6 cm Innendurchmesser, 90 cm Länge) mindestens 6 Stunden lang durchgeführt. Die Reaktionsprodukte werden durch übliche Methoden abgetrennt und charakterisiert. Die quantitative Bestimmung der Reaktionsprodukte und der Ausgangsstoffe erfolgt gaschromatographisch.

Tabelle 1

| Beispiel | Katalysator | Temperatur (°C) | Druck (bar) | Mol-Verhältnis EDA/IBU** | Ausbeute an TBDA* in % der Th. (bez. auf EDA) |
|---|---|---|---|---|---|
| 1 | C | 300 | 300 | 1/2 | 8,6 |
| 2 | C | 300 | 300 | 1/3 | 9,3 |
| 3 | A | 300 | 300 | 1/2 | 14,0 |
| 4 | A | 300 | 300 | 1/5 | 5,9 |
| 5 | B | 300 | 300 | 1/2 | 11,2 |
| 6 | B | 300 | 300 | 1/5 | 20,5 |
| 7 | D | 300 | 300 | 1/2 | 11,1 |

* TBEDA = N-tert.-Butylethylendiamin
** IBU = Isobuten; EDA = Ethylendiamin

## Patentansprüche

**1.** Verfahren zur Herstellung von Diaminen der allgemeinen Formel (I)

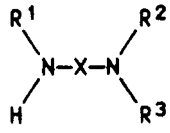

(I)

in der $R^1$, $R^2$ und $R^3$ für Wasserstoff oder eine Alkyl-, Aryl-, Alkoxy-, Aralkyl- oder Cycloalkylgruppe

stehen kann und X eine Gruppierung der Formel (II) beschreibt,

$(CR^4R^5)_m$ (II)

wobei $R^4$ und $R^5$ obige Bedeutung annehmen können wie $R^1$, und m die Werte zwischen 1 und 12 annehmen kann, dadurch gekennzeichnet, daß man Diamine der Formel (III)

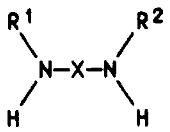

(III)

wobei $R^1$, $R^2$ und X obige Gruppen darstellen, wie in Formel (I) beschrieben, mit Olefinen der Formel (IV)

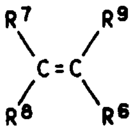

(IV)

in der die Reste $R^6$ bis $R^9$ Wasserstoff oder einen in $\alpha$-Stellung unverzweigten Alkylrest darstellen, in Gegenwart von Zeolithen als Katalysatoren umsetzt.

2. Verfahren nach Anspruch 1, dadurch gekennzeichnet, daß man Ethylendiamin oder Propylendiamin umsetzt.

3. Verfahren nach den Ansprüchen 1 und 2, dadurch gekennzeichnet, daß man als Katalysatoren Zeolithe des Pentasiltyps verwendet.

4. Verfahren nach Ansprüchen 1 bis 3, dadurch gekennzeichnet, daß man als Katalysatoren Aluminosilikatzeolithe des Pentasiltyps verwendet.

5. Verfahren nach den Ansprüchen 1 bis 3, dadurch gekennzeichnet, daß man als Katalysatoren Borsilikatzeolithe des Pentasiltyps verwendet.

6. Verfahren nach den Ansprüchen 1 bis 3, dadurch gekennzeichnet, daß man als Katalysatoren Eisensilikatzeolithe des Pentasiltyps verwendet.

7. Verfahren nach den Ansprüchen 1 und 2, dadurch gekennzeichnet, daß man als Katalysatoren Aluminiumsilikatzeolithe des Faujasittyps verwendet.

8. Verfahren nach den Ansprüchen 1 und 2, dadurch gekennzeichnet, daß man als Katalysatoren Aluminiumsilikatzeolithe des Erionit- und Offretittyps verwendet.

9. Verfahren nach den Ansprüchen 1 bis 8, dadurch gekennzeichnet, daß man Zeolithkatalysatoren verwendet, die mit einem Übergangsmetall und/oder Edelmetall und/oder mit seltenen Erden dotiert sind.

10. Verfahren nach den Ansprüchen 1 bis 8, dadurch gekennzeichnet, daß man Katalysatoren verwendet, die mit Alkalimetallen und/oder Erdalkalimetallen dotiert sind.

11. Verfahren nach den Ansprüchen 1 bis 10, dadurch gekennzeichnet, daß pro mol Amin 0,05 bis 7 mol Olefin eingesetzt werden.

## Claims

**1.** A process for the preparation of a diamine of the formula (I)

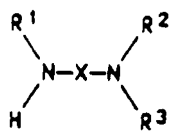

(I)

where $R^1$, $R^2$ and $R^3$ are each hydrogen, alkyl, aryl, alkoxy, aralkyl or cycloalkyl and X is a group of the formula (II)

$(CR^4R^5)_m$ (II)

where $R^4$ and $R^5$ have the meanings given above for $R^1$ and m is from 1 to 12, wherein a diamine of the formula (III)

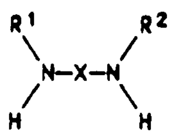

(III)

where $R^1$, $R^2$ and X are the groups described above for formula (I), is reacted with an olefin of the formula (IV)

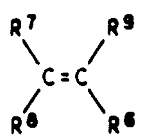

(IV)

where $R^6$ to $R^9$ are each hydrogen or alkyl which is straight-chain in the $\alpha$-position, in the presence of a zeolite as a catalyst.

**2.** A process as claimed in claim 1, wherein ethylenediamine or propylenediamine is reacted.

**3.** A process as claimed in claim 1 or 2, wherein the catalyst used is a zeolite of the pentasil type.

**4.** A process as claimed in any of claims 1 to 3, wherein the catalyst used is an aluminosilicate zeolite of the pentasil type.

**5.** A process as claimed in any of claims 1 to 3, wherein the catalyst used is a borosilicate zeolite of the pentasil type.

**6.** A process as claimed in any of claims 1 to 3, wherein the catalyst used is an iron silicate zeolite of the pentasil type.

**7.** A process as claimed in claim 1 or 2, wherein the catalyst used is an aluminosilicate zeolite of the faujasite type.

**8.** A process as claimed in claim 1 or 2, wherein the catalyst used is an aluminosilicate zeolite of the erionite or offretite type.

**9.** A process as claimed in any of claims 1 to 8, wherein a zeolite catalyst which has been doped with a

transition metal and/or noble metal and/or rare earth metal is used.

**10.** A process as claimed in any of claims 1 to 8, wherein a catalyst which has been doped with alkali metals and/or alkaline earth metals is used.

**11.** A process as claimed in any of claims 1 to 10, wherein from 0.05 to 7 moles of olefin are used per mole of amine.

**Revendications**

**1.** Procédé de préparation de diamines de formule générale (I)

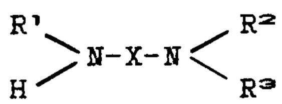

(I)

dans laquelle $R^1$, $R^2$ et $R^3$ peuvent être mis chacun pour un atome d'hydrogène ou pour un groupement alkyle, aryle, alcoxy, aralkyle ou cycloalkyle et X représente un groupement de formule (II)

$(CR^4R^5)_m$

$R^4$ et $R^5$ pouvant avoir les significations données ci-dessus pour $R^1$ et m pouvant avoir les valeurs de 1 à 12, caractérisé en ce qu'on fait réagir, en présence de zéolithes servant de catalyseurs, des diamines de formule (III)

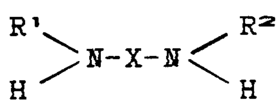

(III)

$R^1$, $R^2$ et X représentant les mêmes groupements que ceux qui ont été définis ci-dessus pour la formule (I), avec des oléfines de formule (IV)

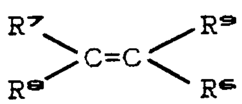

(IV)

dans laquelle les restes $R^6$ à $R^9$ représentent chacun un atome d'hydrogène ou un reste alkyle non ramifié en position $\alpha$.

**2.** Procédé selon la revendication 1, caractérisé en ce qu'on fait réagir de l'éthylènediamine ou de la propylènediamine.

**3.** Procédé selon la revendication 1 ou 2, caractérisé en ce qu'on utilise, comme catalyseur, des zéolithes du type pentasil.

**4.** Procédé selon l'une quelconque des revendications 1 à 3, caractérisé en ce qu'on utilise, comme catalyseurs, des zéolithes d'aluminosilicate du type pentasil.

**5.** Procédé selon l'une quelconque des revendications 1 à 3, caractérisé en ce qu'on utilise, comme catalyseurs, des zéolithes de borosilicate du type pentasil.

**6.** Procédé selon l'une quelconque des revendications 1 à 3, caractérisé en ce qu'on utilise, comme catalyseurs, des zéolithes de ferrosilicate du type pentasil.

7. Procédé selon la revendication 1 ou 2, caractérisé en ce qu'on utilise, comme catalyseurs, des zéolithes d'aluminosilicate du type faujasite.

8. Procédé selon la revendication 1 ou 2, caractérisé en ce qu'on utilise, comme catalyseurs, des zéolithes d'aluminosilicate du type érionite et offretite.

9. Procédé selon l'une quelconque des revendications 1 à 8, caractérisé en ce qu'on utilise des catalyseurs zéolithiques qui sont dopés avec un métal de transition et/ou un métal précieux et/ou avec des terres rares.

10. Procédé selon l'une quelconque des revendications 1 à 8, caractérisé en ce qu'on utilise des catalyseurs qui sont dopés avec des métaux alcalins et/ou des métaux alcalino-terreux.

11. Procédé selon l'une quelconque des revendications 1 à 10, caractérisé en ce qu'on met en réaction, par mole d'amine de 0,05 à 7 moles d'oléfine.